(19) Europäisches Patentamt
European Patent Office
Office européen des brevets

(11) Publication number: **0 188 647 B1**

(12) # EUROPEAN PATENT SPECIFICATION

(45) Date of publication of patent specification: **24.05.89**

(51) Int. Cl.[4]: **C 07 H 19/10,** A 61 K 31/70

(21) Application number: **85100715.3**

(22) Date of filing: **24.01.85**

(54) **Acylated derivatives of cytidine-diphosphate-choline, process for their preparation and their therapeutic use.**

(43) Date of publication of application:
**30.07.86 Bulletin 86/31**

(45) Publication of the grant of the patent:
**24.05.89 Bulletin 89/21**

(84) Designated Contracting States:
**AT BE CH DE FR GB IT LI LU NL SE**

(56) References cited:
**"The Merck Index; An Encyclopedia of Chemicals and Drugs", 9th edition, 1976, page 299, Merck & Co., Inc., Rahway, N.J., US;**

**CHEMICAL ABSTRACTS, vol. 100, no. 25, 18th June 1984, page 645, no. 210323s, Columbus, Ohio, US; M. MARIAN: "Acetyl derivatives of nucleoside 5'-triphosphates" & MICROCHEM. J. 1984, 29(2), 219-27**

(73) Proprietor: **NEOPHARMED S.p.A.**
**Via Pordoi, 18**
**I-20021 Baranzate di Bollate Milano (IT)**

(72) Inventor: **Zappia, Vincenzo**
**Via San Giacomo dei Capri 109**
**I-80100 Napoli (IT)**
Inventor: **De Rosa, Mario**
**Via Nicolardi 188**
**I-80100 Napoli (IT)**

(74) Representative: **Bühling, Gerhard, Dipl.-Chem. et al**
**Patentanwaltsbüro Tiedtke-Bühling-Kinne Grupe-Pellmann-Grams-Struif Bavariaring 4**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 188 647 B1

## Description

This invention concerns novel derivatives of cytidine-diphosphate-choline having the general formula:

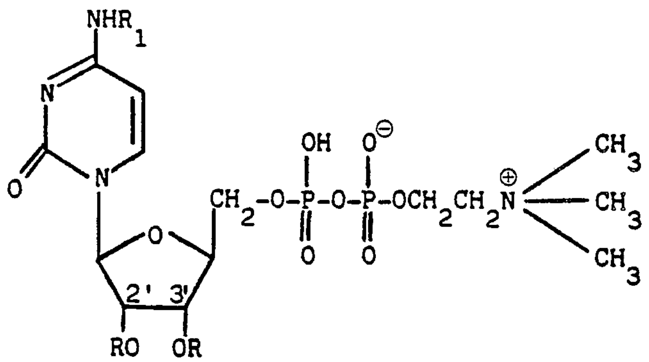

(I)

in which R represents an acyl radical chosen from the group comprised of monocarboxylic saturated and unsaturated, linear or branched, fatty acids having from 3 to 7 carbon atoms, and $R_1$ represents H or an acyl radical identical to R.

Cytidine-diphosphate-choline, referred to from now on as the abbreviation CDP-choline, represents the active form of choline and is a key intermediate in the biosynthesis of complex lipids. The importance of the cytidinic nucleotides in the formation of diester bonds in phospholipid molecules has been broadly documented in fact.

As shown in the literature, the lecithins and sphingomyelins are formed by means of a reaction catalysed by microsomal enzymes, in which the CDP-choline donates the phosphorylcholinic fragment to the D-α,β-diglyceride or to the N-acylsphingosine respectively.

Furthermore one should remember that CDP-choline acts as a P-choline donor to 1-alkyl, 1'-enyl-2-acyl-*sn*-glycerol, forming the plasmalogens.

CDP-choline is biosynthetized from P-choline, and CTP, by means of the enzyme choline phosphate-cytidine transferase. This enzymic activity has been found in the particle free fraction of the cytoplasm and in the microsomal fraction. It is appropriate to underline, with reference to this, that the formation of CDP-choline represents the slowest step and therefore the limitative one in the whole metabolic pathway. The cellular concentrations of this metabolite therefore have a critical importance in the regulation of the biosynthesis of phospholipids.

As a consequence of its biochemical roles, CDP-choline finds a use and pharmacological indication prevailingly in a series of alterations in the central nervous system. In this organ, in fact, the structural and functional integrity of phospholipid membranes is particularly critical. The form of administration used up to now has been parenteral. The pharmacological activity of the molecule has been demonstrated in various diseases such as the sequences of cerebral apoplexy, different types of cerebral ischaemia, Parkinson's disease, cranial fractures and their sequences.

This invention concerns the use of the acylated analogues of CDP-choline, as previously given in formula I, in therapy.

They are obtained by acylation at positions $N_4$ and/or 2', 3' of the molecule with the already defined carboxylic acids, in particular and preferably monocarboxylic acids, naturally present in mammals, of different chain lengths, linear or branched, saturated or unsaturated.

Esterification concerns some or all of the reactive positions on the molecule mentioned previously.

The acylated derivatives of CDP-choline are sufficiently stable in acid or alkaline environments so as not to be chemically broken down by the variations in pH associated with the journey through the gastrointestinal tract.

The presence of strongly hydrophobic chains significantly modifies the molecular morphology, impeding, both at the gastric level and at the intestinal level, hydrolysis of the drug at the pyrophosphoric bond. This is possible only after removal of the acylic groups in sequence by the action of the gastric and intestinal acyl esterases and/or amidases and peptidases.

Drug absorption is conditioned by the hydrolysis of the acyl groups and therefore acylation of CDP-choline conditions the absorption of the cholinic and cytosinic components of the drug.

The acyl residues that are released can be considered as metabolites normally present in the organism with absolutely negligible toxicity levels.

The $N_4$,2',3'-triacyl derivatives of CDP-chloine administered orally act as retard forms of CDP-choline. The consequence of this process of slow release of CDP-choline in the organism is more uniform and gradual distribution of the active principle.

As already indicated, according to this invention the CDP-choline derivatives have, in oral form, indications analogous to those for CDP-choline.

More simply, according to the invention, oral use of the CDP-choline derivatives is forseen in table form, each containing from 200 to 1500 mg of the active principle, to be administered 2—3 times a day in

the following illnesses:

arteriosclerosis especially cerebral,
short and long term treatment of cerebrovascular accidents,
short and long term treatment of the consequences of a stroke,
treatment of Parkinson's and Parkinson-like syndromes, in particular in the arteriosclerotic form,
anti-depression treatment,
treatment of cerebral traumatic coma,
prevention and therapy of hyaline membrane disease (IRDS),
therapy in acute and chronic hepatitis (viral hepatitis etc.),
therapy and prevention of fatty liver in alcoholics,
coadjuvant therapy in liver cirrhosis.

This invention also concerns the preparation of the derivatives of CDP-choline of formula I. More specifically, the process for the preparation of the derivatives (I) form another object of this invention, bound to the type of functionalisation or esterification desired. Therefore:

1) In N-monoacylation of CDP-choline at the aminic group of the carbon 4 of the pyrimidinic nucleus, the process, according to this invention, is characterized by the fact that the tetrabutylammonium salt of CDP-choline is reacted with an excess of imidazolide of the desired carboxylic acid in an aprotic dipolar solvent (DMF, formamide or pyridine, of which DMF represents the best one), at a temperature of 50°C for 36 hours and in the presence of an acylation catalyst such as 4-(N,N-dimethylamino)pyridine.

The imidazolide of the carboxylic acid is in turn prepared from the carboxylic acid by reaction with N,N'-carbonyldiimidazole in a solution of anhydrous DMF.

2) In the realisation of $N^4$-acyl-2',3'-di-O-acyl-CDP-choline one can use the same method described in section 1 above, obviously using an excess of imidazolide and, as a necessity, prolonging the time of the reaction to 96 hours, at a reaction temperature of 50°C.

3) Acylation with an aqueous mixture of poorly solvating compounds like THF, acetone, acetonitrile, the acylation takes place exclusively at the hydroxyl groups of ribose.

The best yields are obtained using mixtures $H_2O$/THF or $H_2O$/acetonitrile (1:4 v/v) as solvents. The acylation reaction never runs to completion and the best yields are obtained using a molar ratio of 2:1 between imidazolide (as prepared separately in anhydrous THF) and CDP-choline.

Under these conditions monoacylation of CDP-choline takes place exclusively, unselectively at one of the free hydroxyl groups at 2' or 3' of the ribose, giving 2'(3')-O-acyl-CDP-choline (Formula II).

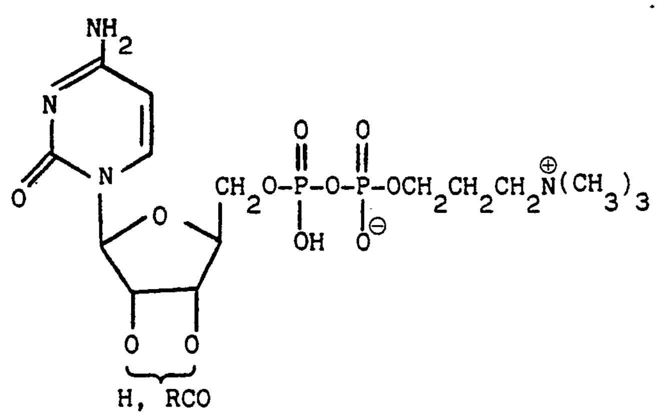

(II)

The chromotographic purification of the reaction mixture on DOWEX AG 1:8 does not allow for resolution of the two isomers of CDP-choline acylated at 2' and 3' of the ribose respectively.

Therefor all the spectroscopic characterizations were carried out on the mixture of the two isomers that are stable in aqueous solution at pH 6.5 or lyophilized.

The identification of all the monoacyl and triacyl derivatives of CDP-choline is essentially based on the $^1H$—NMR and UV spectroscopic data.

The $^1H$—NMR data concisely reported in Table 1 refer to the derivatives of valeric acid and are consistent with the structural assignations for each derivative (see Table 1).

TABLE 1

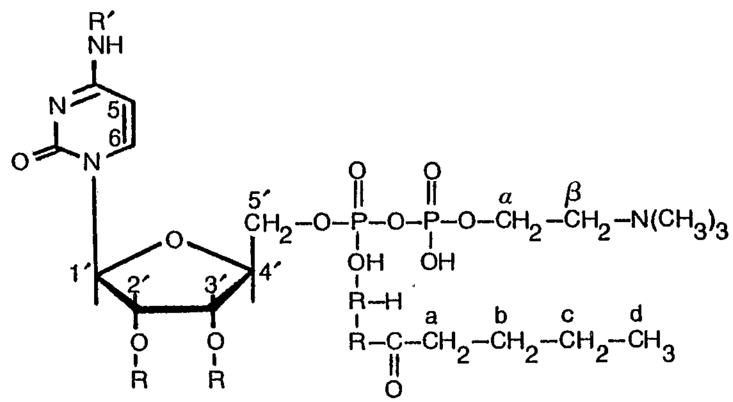

| Assignation | 6 | 5 | 1′ | 2′ | 3′ | 4′ | 5′ | α | β | N—C³H | a | b | c | d |
|---|---|---|---|---|---|---|---|---|---|---|---|---|---|---|
| Compound | Chemical shifts in δ | | | | | | | | | | | | | |
| 1 | 8.16 d | 6.27 d | 5.88 d | 4.41—4.20 c | | | | | 3.69 s(wide) | 3.22 | / | / | / | / |
| 2 | " | " | 6.12 c | 5.38—5.46 c | | 4.68—4.39 c | | | " | " | 2.49 t | 1.62 q | 1.3 se | 0.89 t |
| 3 | 8.42 d | 7.42 d | 5.95 d | 4.45—4.20 c | | | | | " | " | " | " | " | " |
| 4 | " | " | 6.21 d | 5.50—5.48 c | | 4.62 ABX | 4.22—4.33 ABX | 4.40 s (wide) | " | " | " | " | " | " |

Table 1 — ¹H—NMR data for CDP-choline acylated derivatives. The spectra were obtained in $^2H_2O$ using a Bruker 500 MHz spectrometer. The chemical shifts are given in δ.
1) CDP-choline; 2) 2′(3′)-O-valeryl-CDP-choline; 3) N⁴-valeryl-CDP-choline; 4) N⁴-valeryl-2′,3′-di-O-valeryl-CDP-choline.
se (sestet), q (quintet); t (triplet); d (duplet); s (singlet); c (complex signal)

The nuclear magnetic resonance spectrum in the case of 2′(3′)-O-valeryl CDP-choline shows that only one acyl group has been introduced and is localised at the level of the 2′ or 3′ hydroxyl groups of ribose. One can in fact see a shift to lower fields on the part of the complex signal to 4,0—4,5, attributable to the H's on the hydroxylic group carrying carbons, whereas the proton's signal on the carbons 5 and 6 of the cytosinic nucleus are found at the same values of chemical shift that they have in CDP-choline thus excluding the possibility of an acylation of the aminic group at the carbon 4 of the aromatic system.

In the spectrum of $N^4$-valeryl CDP-choline, however, the presence of a single unit of valeric acid per molecule is observed, specifically localised at the nitrogen in position 4 of the cytosinic system. In fact while the characteristic shift to lower fields of protons 5 and 6 of the chromophore is revealed, the ribose protons show resonance in the same fields as CDP-choline.

Finally, in the $N^4$-valeryl-2′,3′-di-O-valeryl-CDP-choline spectrum, the integration of the protons of the acylating group shows the presence of three units of valeric acid per molecule of CDP-choline. The total acylation of the molecule leads to a shift to low fields both of the aromatic protons and of the hdyrocarbons of the ribose.

The examples that follow illustrate, without in any way limiting, the processes according to this invention.

## Example 1

Synthesis of 3′(2′)-O-valeryl-CDP-choline

The chemical synthesis of CDP-choline acylated with valeric acid at the hydroxyl 2′ or 3′ of ribose, is carried out by condensation of CDP-choline with the imidazolide of valeric acid.

The latter, which is obtained by a reaction between valeric acid and N,N′-carbonyldiimidazole in a rigorously anhydrous environment, permits the acylation to be selectively carried out, in mild conditions and in an aqueous-organic environment, on one of the two ribose hydroxyl groups.

In a standard procedure, the preparation of the valeric imidazolide is carried out by reacting 1.3 g (8 mmoles) of carbonyldiimidazole with 500 mg (4.9 mmoles) of valeric acid, 5 mls of tetrahydrofurane, anhydrified on molecular sieves and kept under anhydrous nitrogen. The reaction, carried out in nitrogen in very anhydrous conditions, is completed in about 10 minutes.

Like results are obtained if, while keeping the volume of solvent unaltered, double or triple quantities of the reactants are used or if the THF is substituted with acetonitrile or anhydrous acetone.

The acylation of CDP-choline is carried out in an aqueous-organic environment by mixing under stirring at room temperature 20 ml of a solution of 250 mM CDP-choline with 5 ml of the organic solution of imidazolide at different concentrations (1.6, 3.2, 4.8 M). In every case the reaction mixture initially appears like a stable white coloured emulsion; it becomes clear as the acylation of CDP-choline takes place. In about 4 hours the reaction reaches maximum yield. Working at 70°C the course of the reaction is much quicker (2h) but the yield of acylated product is not significantly modified. The 3′(2′)-O-valeryl-CDP-choline can be isolated by evaporation of the solvent in a vacuum with a stream of nitrogen. The remaining aqueous solution is brought to pH 8.5 with 0.5N KOH and is then adsorbed onto a DOWEX 1 × 8 (formiate) column.

The elution is carried out first with $H_2O$ and then with a linear gradient of formic acid from 0.00 to 0.02 M.

The 3′(2′)-O-valeryl-CDP-choline has a very similar UV spectrum to that of CDP-choline, showing a maximum of 280 nm ($=12.8 \times 10^3$) in water.

The information that one can deduce from the analysis of the NMR spectrum of $^{13}C$ fully confirms the structural identity of the molecule, excluding once again that the aminic group on the aromatic system has been involved in the acylation reaction.

In fact the cytosinic carbon signals, as in CDP-choline, have chemical shifts of 160.49 δ ($C_2$) 168.98 δ ($C_4$), 99.29 δ ($C_5$), and 144.28 δ ($C_6$) respectively. The ribose carbons however give rise to a good ten signals, five for each of the 2 isomers respectively acylated in positions 2′ or 3′, while the cholinic carbons are found at the same chemical shifts as in CDP-choline. On the other hand, at high fields one finds the aliphatic carbons of the valeric acid.

Structural analogues of CDP-choline, acylated at 2′ or 3′ with fatty acids from $C_3$ to $C_7$, saturated or unsaturated, linear or branched, can be obtained according to the same reaction scheme given in Example 1, substituting the valeric acid with the desired acid.

## Example 2

Synthesis of $N^4$-valeryl-CDP-choline

0.2 mmoles of tetrabutylammonium hydroxide (1 ml 0.2 M) are added to 100 mg of CDP-choline, dissolved in 5 ml of $H_2O$, and then one proceeds to the lyophilisation of the sample. The tetrabutylammonium salt (TEBA) of CDP-choline is then dissolved in 4 ml of anhydrous DMF containing 20 mg of 4-dimethylaminopyridine.

2 mmoles of the imidazolide of valeric acid, obtained by reacting 204 mg of acid dissolved in 1 ml of anhydrous DMF with 324 mg of N,N′-carbonyl-diimidazole, are added to the resulting solution.

The reaction, stirred for 36 hours at 50°C, is interrupted by removing the solvent in a vacuum and the oleaginous residue obtained is triturated three times with ethyl acetate.

The reaction mixture is purified by chromatography on silica, by elution with $H_2O$ in $CH_3OH$ in a linear

gradient from 0 to 20% v/v. Th $N^4$-valeryl-CDP choline is obtained with a yield of 50—60% and $N^4$-valeryl-2′,3′-di-O-valeryl-CDP-choline, $N^4$-acylated with fatty acids from $C_3$ to $C_7$, saturated or unsaturated, linear or branched, can be obtained according to the method given in this example, substituting the valeric acid with the acid desired.

Example 3

Synthesis of $N^4$-valeryl-2′,3′-di-O-valeryl-CDP-choline

One proceeds as described in the former example up to the preparation of the solution of the TEBA salt of CDP-choline in DMF, containing 4-(N,N-dimethylamine)pyridine.

At time nought and after 48 hours, 2 mmoles of the imidazolide of valeric acid, obtained by reacting 204 mg of the acid dissolved in 1 ml of anhydrous DMF with 324 mg of N,N′-carbonyl-diimidazole, are added to this solution. The reaction mixture is stirred for 96 hours at 50°C, and the reaction is interrupted by removing the solvent in a vacuum and triturating the oleaginous residue obtained three times with ethyl acetate.

The triacylate (105 mg) is isolated pure by chromatography on a silica column by elution with $H_2O$ in $CH_3OH$ in a linear gradient from nought to 20% v/v.

Structural analogues of CDP-choline, triacylated with fatty acids from $C_3$ to $C_7$, saturated or unsaturated, linear or branched, can be obtained according to the same method given in Example 3, substituting the valeric acid with the acid desired.

The pharmacokinetic characteristics of $N^4$-valeryl-2′,3′-di-O-valeryl-CDP-choline have been defined in rats by oral administration of the product with a double label of $^3H$ at 5′ on the cytosinic nucleus and $^{14}C$ on the cholinic methyls. The required data refer to the distribution of radioactivity in the animal and also to structural identity of the radioactive molecular species present in some of the more significant biological samples (liver, brain, faeces, gastric and intestinal contents).

A precise analysis of the role played by the functionalisation in the absorption process and metabolism of the triacylated drug has imposed a comparison of the obtained data with those of a control experiment, in which rats were given orally equimolecular doses of (5-$^3$H; methyl-$^{14}$C) CDP-choline. The caracterization of the labelled metabolites present in the liver, brain, faeces, gastric and intestinal contents of the animals treated orally with pharmacological doses (20 μmoles/kg) of (5$^3$-H; methyl-$^{14}$-C)-CDP-choline or of $N^4$-valeryl-2-2′,3′-di-O-valeryl-(5-$^3$H; methyl-$^{14}$C)-CDP-choline leads to their preventive extraction from the tissues.

The scheme of extraction consents the recovery of three different classes of labelled metabolites:

1) Hydrosoluble metabolites, subsequently analysed by HPLC.

2) Metabolites with high molecular weights, proteins and nucleic acids, recovered by thermal denaturation in a hydroalcoholic environment.

3) Lipids, subsequently purified by chromatography on silica.

The experimental results show that $N^4$-valeryl-2′,3′-di-O-valeryl CDP-choline is an interesting oral form of slow release of CDP-choline.

The global analysis of the metabolic picture, as compared to that found in the experiment with CDP-choline, substantially shows a better and more gradual distribution of the radioactivity in the organism. The metabolic pictures of the organs examined (liver and brain) were substantially equal and acylated cytosinic metabolites were not found. This indicates that the valeric acid chains are quantitatively removed before the cytosinic component is used which does not appear to be significantly compromised in comparison with the control experiment.

The bioavailablility in terms of recovery of the structural components of the drug in 24 hours is very similar for CDP-choline and its triacylated analogue, the faecal and urinary excretions being quite comparable.

The radioactive levels and the structural identity of the labelled species present at gastric and intestinal level, clearly show the notable consistency of the protective effect exercised by the valeric acid residues as regards the pyrophosphatases, intestinal especially, and the indirect control operated on the speed of absorption.

In fact, while after 30 minutes in the control experiment at intestinal level not more than 5% of the radioactivity present in the dose and only 0.2% of the double labelled CDP-choline administered is found, on the other hand in the case of trivaleryl-CDP-choline 29% of the variably acylated double labelled molecule is still found after an hour and a total radioactivity equal to 30% of that present in the administered dose is detected.

To confirm this datum, in the plasmatic component and in the organs, the maximum uptake is definitely moved to longer periods of time (5 hours) with trivaleryl-CDP-choline.

**Claims**

1. Derivatives of CDP-choline having the formula I:

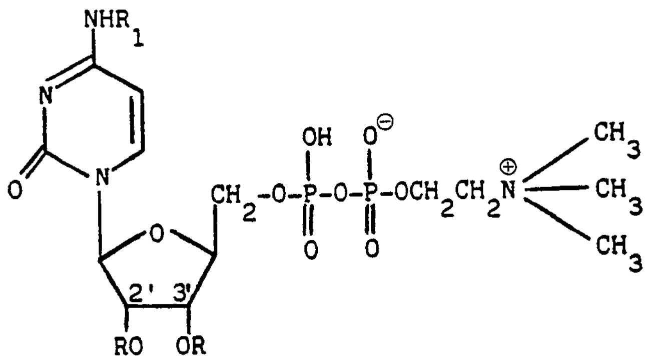

(I)

in which two of the $R_1$ and R groups represent hydrogen and the third one represents an acyl radical of a saturated or unsaturated, linear or branched, monocarboxylic fatty acid having from 3 to 7 carbon atoms, or all the $R_1$ and R groups represent identical acyl radicals.

2. A process for the preparation of the O-monoacylated derivatives of CDP-choline of formula I according to claim 1, wherein $R_1 = H$, characterized by reacting CDP-choline with the acylimidazole (imidazolide) of a saturated or insaturated, linear or branched monocarboxylic fatty acid containing 3 to 7 carbon atoms in an aqueous mixture of a poorly solvating compound.

3. A process according to claim 2, characterized in that said poorly solvating compound is selected among tetrahydrofuran, acetone and acetonitrile.

4. A process according to claim 3, characterized in that the reaction medium is a mixture 1:4 v/v of water and the poorly solvating compound.

5. A process according to claim 2, characterized in that the molar ratio between imidazolide and CDP-choline is 2:1.

6. A process for the preparation of $N^4$-monoacylated derivatives of formula I according to claim 1 in which $R_1$ = acylic radical and R = H, characterized in that the tetrabutylammonium salt of CDP-choline, in a homogeneous solution of an aprotic dipolar solvent and in the presence of an acylation catalyst, is reacted with the imidazolide of a saturated or insaturated, linear or branched monocarboxylic fatty acid containing 3 to 7 carbon atoms to be introduced at $N^4$.

7. A process according to claim 6, characterized in that said solvent is selected among formamide, pyridine and dimethylformamide.

8. A process according to claim 6, characterized in that said acylation catalyst is 4-N,N-dimethylaminopyridine.

9. A process according to claim 6, characterized in that the reaction is carried out with an excess of imidazolide.

10. A process according to claim 6, characterized in that the reaction is carried out at 50°C and for 36 hours.

11. A process for the preparation of $N^4$-acyl-2',3'-di-O-acyl derivatives of CDP-choline of formula I according to claim 1, characterized in that the reaction is carried out according to claim 6, with an excess of imidazolide, for a time of 96 hours and at a temperature of 50°C.

12. Pharmaceutical composition characterized by containing as the active ingredient, a derivative of CDP-choline, according to claim 1, together with the usual excipients and carriers.

13. Pharmacological composition according to claim 12, characterized by the fact of being in a form suitable for oral administration.

14. Pharmaceutical composition according to claim 12, useful for the treatment of cerebral apoplexy, cerebral ischaemia, Parkinson's disease, cranial traumatology.

15. Pharmaceutical composition according to claims 12 and 13, characterized by containing 200 to 1.500 mg of the active principle, as a unitary dosage.

**Patentansprüche**

1. Derivate von CDP-Cholin mit der Formel I:

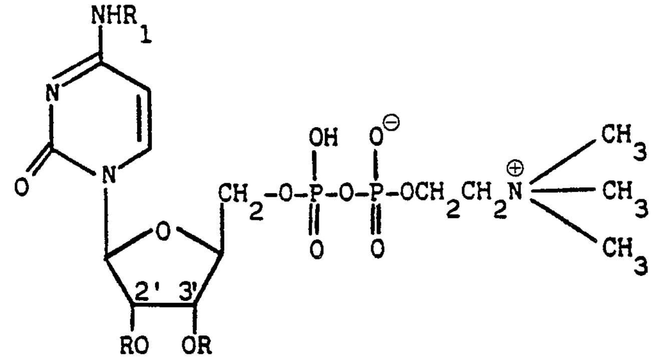

(I)

worin zwei der Gruppen $R_1$ und R Wasserstoff bedeuten und die dritte einen Acylrest einer gesättigten oder ungesättigten, linearen oder verzweigten einwertigen Fettsäure mit 3 bis 7 Kohlenstoffatomen bedeutet oder worin alle Gruppen $R_1$ und R identische Acylreste bedeuten.

2. Verfahren zur Herstellung der O-monoacylierten Derivate von CDP-Cholin mit der Formel I gemäß Anspruch 1, worin $R_1$ = H, dadurch gekennzeichnet, daß CDP-Cholin in einer wäßrigen Mischung einer schlecht solvatisierenden Verbindung mit dem Acylimidazol (Imidazolid) einer gesättigten oder ungesättigten, linearen oder verzweigten einwertigen Fettsäure, die 3 bis 7 Kohlenstoffatome enthält, umgesetzt wird.

3. Verfahren nach Anspruch 2, dadurch gekennzeichnet, daß die schlecht solvatisierende Verbindung aus Tetrahydrofuran, Aceton und Acetonitril ausgewählt ist.

4. Verfahren nach Anspruch 3, dadurch gekennzeichnet, daß das Reaktionsmedium eine 1:4-Mischung (Vol./Vol.) aus Wasser und der schlecht solvatisierenden Verbindung ist.

5. Verfahren nach Anspruch 2, dadurch gekennzeichnet, daß das Molverhältnis zwischen Imidazolid und CDP-Cholin 2:1 beträgt.

6. Verfahren zur Herstellung von $N^4$-monoacylierten Derivaten der Formel I gemäß Anspruch 1, worin $R_1$ = Acylrest und R = H, dadurch gekennzeichnet, daß das Tetrabutylammoniumsalz von CDP-Cholin in einer homogenen Lösung eines aprotischen, dipolaren Lösungsmittels und in Gegenwart eines Acylierungskatalysators mit dem Imidazolid einer gesättigten oder ungesättigten, linearen oder verzweigten einwertigen Fettsäure, die 3 bis 7 Kohlenstoffatome enthält, zur Einführung am $N^4$ umgesetzt wird.

7. Verfahren nach Anspruch 6, dadurch gekennzeichnet, daß das Lösungsmittel aus Formamid, Pyridin und Dimethylformamid ausgewählt ist.

8. Verfahren nach Anspruch 6, dadurch gekennzeichnet, daß der Acylierungskatalysator 4-N,N-Dimethylaminopyridin ist.

9. Verfahren nach Anspruch 6, dadurch gekennzeichnet, daß die Umsetzung mit einem Überschuß des Imidazolids durchgeführt wird.

10. Verfahren nach Anspruch 6, dadurch gekennzeichnet, daß die Umsetzung bei 50°C und 36 h lang durchgeführt wird.

11. Verfahren zur Herstellung von $N^4$-Acyl-2',3'-di-O-acylderivaten von CDP-Cholin der Formel I gemäß Anspruch 1, dadurch gekennzeichnet, daß die Umsetzung nach Anspruch 6 mit einem Überschuß des Imidazolids 96 h lang und bei einer Temperatur von 50°C durchgeführt wird.

12. Arzneimittel, dadurch gekennzeichnet, daß es als Wirkstoff ein Derivat von CDP-Cholin nach Anspruch 1 zusammen mit den üblichen Bindemitteln und Trägern enthält.

13. Arzneimittel nach Anspruch 12, dadurch gekennzeichnet, daß es in einer zur oralen Verabreichung geeigneten Form vorliegt.

14. Arzneimittel nach Anspruch 12, das sich zur Behandlung von Schlagenfall, zerebraler Ischämie, Parkinsonscher Krankheit und Schädel- bzw. Kopfverletzungen eignet.

15. Arzneimittel nach Ansprüchen 12 bis 13, dadurch gekennzeichnet, daß es als Einzeldosis 200 bis 1500 mg des Wirkstoffs enthält.

**Revendications**

1. Dérivés de CDP-choline répondant à la formule I:

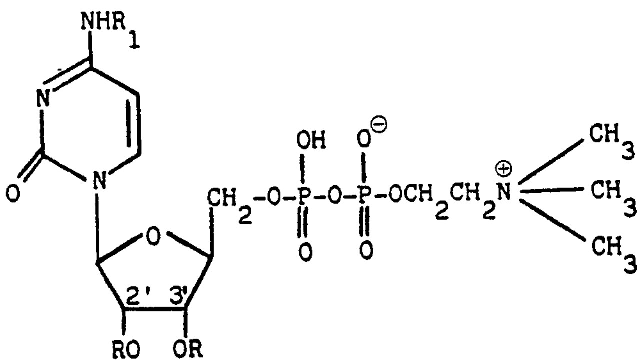

(I)

dans laquelle deux des groupes $R_1$ et R représentent de l'hydrogène et les troisième représente un radical acyle d'un acide gras monocarboxylique, linéaire ou ramifié, saturé ou insaturé, comportant de 3 à 7 atomes de carbone, ou bien tous les groupes $R_1$ et R représentent des radicaux acyle identiques.

2. Procédé de préparation des dérivés O-monoacylés de CDP-choline de formule I suivant la revendication 1, dans laquelle $R_1$ = H, caractérisé en ce que l'on fait réagir de la CDP-choline avec l'acylimidazole (imidazolide) d'un acide gras monocarboxylique linéaire ou ramifié, saturé ou insaturé contenant 3 à 7 atomes de carbone dans un mélange aqueux d'un composé faiblement solvatant.

3. Procédé suivant la revendication 2, caractérisé en ce que le composé faiblement solvatant est choisi parmi le tétrahydrofuranne, l'acetoné et l'acétonitrile.

4. Procédé suivant la revendication 3, caractérisé en ce que le mileau réactionnel est un mélange 1/4 volume/volume d'eau et du composé faiblement solvatant.

5. Procédé suivant la revendication 2, caractérisé en ce que le rapport molaire entre l'imidazole et la CDP-choline est de 2/1.

6. Procédé de préparation de dérivés $N^4$-monoacylés de formule I suivant la revendication 1, dans laquelle $R_1$ = radical acylique et R = H, caractérisé en ce que l'on fait réagir le sel de tétrabutylammonium de CDP-choline, dans une solution homogène d'un solvant bipolaire neutre et en présence d'un catalyseur d'acylation, avec l'imidazolide d'un acide gras monocarboxylique, linéaire ou ramifié, saturé ou insaturé, contenant 3 à 7 atomes de carbone à introduire en $N^4$.

7. Procédé suivant la revendication 6, caractérisé en ce que le solvant est choisi parmi le formamide, la pyridine et le diméthylformamide.

8. Procédé suivant la revendication 6, caractérisé en ce que le catalyseur d'acylation est la 4-N,N-diméthylaminopyridine.

9. Procédé suivant la revendication 6, caractérisé en ce que la réaction est réalisée avec un excès d'imidazolide.

10. Procédé suivant la revendication 6, caractérisé en ce que la réaction est réalisée à 50°C et pendant 36 heures.

11. Procédé de préparation de dérivés $N^4$-acyl-2',3'-di-O-acylés, de CDP-choline de formule I suivant la revendication 1, caractérisé en ce que la réaction est réalisé suivant la revendication 6, avec un excès d'imidazolide, pendant une période de temps de 96 heures et à une température de 50°C.

12. Composition pharmaceutique, caractérisé en ce qu'elle contient, comme ingrédient actif, un dérivé de CDP-choline suivant la revendication 1, en même temps que les excipients et supports usuels.

13. Composition pharmacologique suivant la revendication 12, caractérisée en ce qu'elle est sous une forme appropriée à l'administration orale.

14. Composition pharmaceutique suivant la revendication 12, utilisable pour le traitement de l'apoplexie cérébrale, de l'ischémie cérébrale, de la maladie de Parkinson, de la traumatologie crânienne.

15. Composition pharmaceutiqe suivant l'une ou l'autre des revendications 12 et 13, caractérisée en ce qu'elle contient 200 à 1500 mg de principe actif, comme dosage unitaire.